# EUROPEAN PATENT SPECIFICATION

(11) **EP 3 107 502 B1**
(45) Date of publication and mention of the grant of the patent: **03.06.2020**
(21) Application number: 14802422.7
(22) Date of filing: 21.11.2014
(51) Int. Cl.: A61F 2/38, A61F 2/30

(54) **DEVICE FOR PROTECTING THE POSTERIOR CRUCIATE LIGAMENT IN KNEE PROSTHESES**
VORRICHTUNG ZUM SCHUTZ DES HINTEREN KREUZBANDES IN KNIEPROTHESEN
DISPOSITIF POUR LA PROTECTION DU LIGAMENT CROISE POSTERIEUR DANS DES PROTHESES DU GENOU

(30) Priority: 21.02.2014 IT MI20140254
(43) Date of publication of application: 28.12.2016
(73) Proprietor: Adler Ortho S.p.a., 20032 Cormano (IT)
(72) Inventor: BOISGARD, Stephane, 63000 Clermont Ferrand (FR)
(74) Representative: Modiano, Micaela Nadia
(86) International application number: PCT/EP2014/075261
(87) International publication number: WO 2015/124226

(56) References cited:
- WO-A1-2004/069104
- WO-A1-2011/150238
- US-A1- 2011 251 695
- US-B1- 7 628 817

## Description

The present invention relates to a device for protecting the posterior cruciate ligament in artificial knee prostheses. More particularly, the invention relates to a device for protection against rupture of the posterior cruciate ligament in artificial knee prostheses and for reducing damage if, despite all the protections that might be used, rupture of said posterior cruciate ligament occurs.

As is known, the knee is the most important and complex joint of the lower limb of humans. Although it has a wide range of movement, it has good stability thanks to the presence of ligaments. In the knee it is possible to identify a bone component and a capsuloligamentous apparatus with tendon structures. The bone part is constituted by:
- the lower end of the femur, represented by the trochlea, which comprises the two condyles separated by the intercondylar groove.
- the upper end of the tibia, with its flat internal and external plateaus.
- the patella, arranged at the front.
- two fibrocartilagineous structures, arranged in a semicircle and known as menisci, are interposed between the femoral condyles and the plateaus; their function is to increase articular shape match during the flexion-extension and rotation movement of the knee.

The capsuloligamentous apparatus allows control of the joint flexion and rotation axes by means of a stabilization of the bone components.
- The central joining system or central pivot, located in the intercondylar groove, which is constituted by the two anterior and posterior cruciate ligaments.
- The two cruciate ligaments are fundamental for the stability in the anteroposterior direction (i.e., on the sagittal plane) of the knee.

From what has been described above the considerable importance of cruciate ligaments in giving stability to the knee, which is one of the main characteristics required of a prosthesis for the artificial articulation of the knee, is evident.

Since the anterior cruciate ligament during physical activities is the one most subject to ruptures, and normally it is not recommended to replace it in subjects older than 45 years of age, accordingly:
- the patients that benefit from an artificial knee prosthesis are in most cases without a functional anterior cruciate ligament.
- this means that the posterior cruciate ligament remains the only natural organ that helps to give stability to the artificial knee prosthesis.

If it seems to the orthopedic surgeon, during the operation, that the conditions of the posterior cruciate ligament are such as to ensure the performance required from an artificial knee prosthesis, he will choose to use a prosthesis for protection of the cruciate ligament, known as cruciate retaining prosthesis.

If instead the orthopedic surgeon realizes that the conditions of the posterior cruciate ligament are not sufficient to ensure correct performance for an artificial knee prosthesis, he will choose to use a prosthesis of the PS (posterior stability) type.

In artificial knee prostheses of the CR type, the posterior cruciate ligament therefore has a fundamental role for knee stability and any rupture thereof, which is in any case possible, in addition to rendering the knee certainly unstable, might lead to the need for a new operation to replace the prosthesis with another one more suitable to the new situation that has arisen.
WO 2004/069104, WO 2011/150238, US 2011/251695 and US 7628817 disclose a device for protecting the posterior cruciate ligament in knee prosthesis.

The aim of the present invention is to provide a device for protecting the posterior cruciate ligament in artificial knee prostheses that allows rendering the rupture of the posterior cruciate ligament as unlikely as possible.

Within this aim, an object of the present invention is to provide a device for protecting the posterior cruciate ligament in artificial knee prostheses that allows rendering the assembly constituted by the tibia, the insert, the femur sufficiently shape-matched and dislocation-proof, for an acceptable stability of the knee even in case of rupture of the posterior cruciate ligament.

Another object of the present invention is to provide a device for protecting the posterior cruciate ligament in artificial knee prostheses that has such refinements as to allow the device to be used advantageously to avoid dislocation of the knee even in conditions in which the posterior cruciate ligament is ruptured.

Another object of the present invention is to provide a device for protecting the posterior cruciate ligament in artificial knee prostheses that is highly reliable, relatively simple to provide and has competitive costs.

This aim, as well as these and other objects that will become more apparent hereinafter, are achieved by a device for protecting the posterior cruciate ligament in knee prostheses, as defined in claim 1.

Further characteristics and advantages of the invention will become more apparent from the description of preferred but not exclusive embodiments of the device according to the present invention, illustrated by way of non-limiting example in the accompanying drawings, wherein:
Figure 1 is a top plan view of the protection device according to the invention in its variant for the right knee;
Figure 2 is a top plan view of the protection device according to the invention in its variant for the left knee;
Figure 3 is a perspective view of the device according to the invention;
Figure 4 is a transverse sectional view of the device according to the invention; and
Figure 5 is a longitudinal sectional view of the device according to the invention.

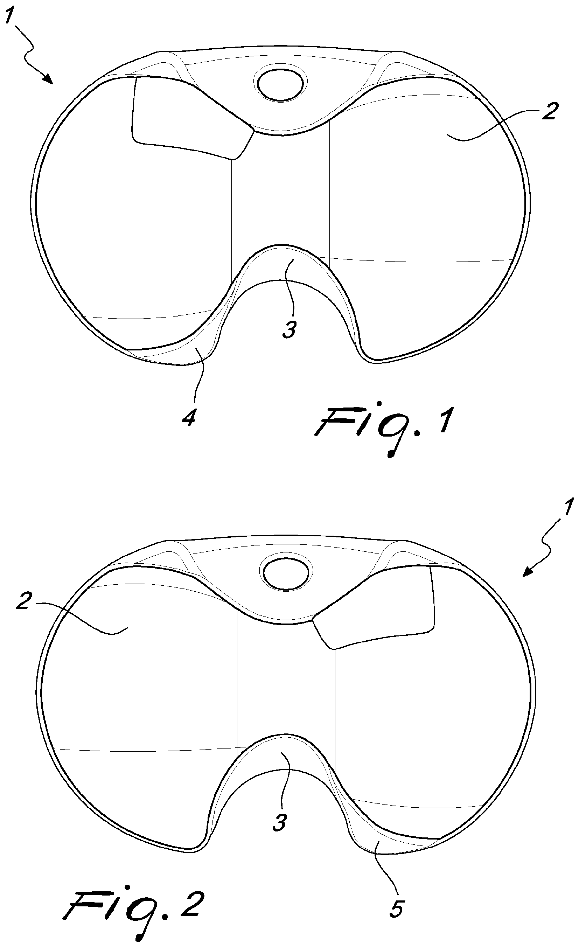

With reference to the figures, the device for protecting the posterior cruciate ligament in an artificial knee prosthesis, designated generally by the reference numeral 1, comprises a body 2 that has a different shape depending on whether it is to be used for a right knee, as shown in Figure 1, or a left knee, as shown in Figure 2.

The body 2 has a substantially elliptical shape, with a hollow or recess 3 with rounded edges, formed at the inner side of the central portion of the body 2.

Conveniently, in the case for example of devices used for the right knee, the hollow 3 has a rounded left edge 4 and substantially an easy passage of the posterior cruciate ligament toward the internal bone of the medial femoral condyle.

By symmetry, the body 2 adapted to be used in the left knee, as shown in Figure 2, has the same recess 3, with a rounded and flattened right edge 5, again to allow the easy passage of the posterior cruciate ligament toward the internal bone of the medial femoral condyle.

Essentially, the posterior cruciate ligament, being inserted in the rear part of the tibial bone and being connected at the other end to the inside of the medial femoral condyle, in order to be rendered safe against any stresses that might lead to its rupture, must use dedicated devices, i.e., right-side devices for right joints and left-side devices for left joints, or anatomically contoured tibiae and inserts.

Conveniently, the body 2 of the device according to the invention, in order to be able to remain functional even if the posterior cruciate ligament ruptures, is provided so that the medial front lip and the lateral front lip are at least 11 mm thicker than the thinner point of the body 2.

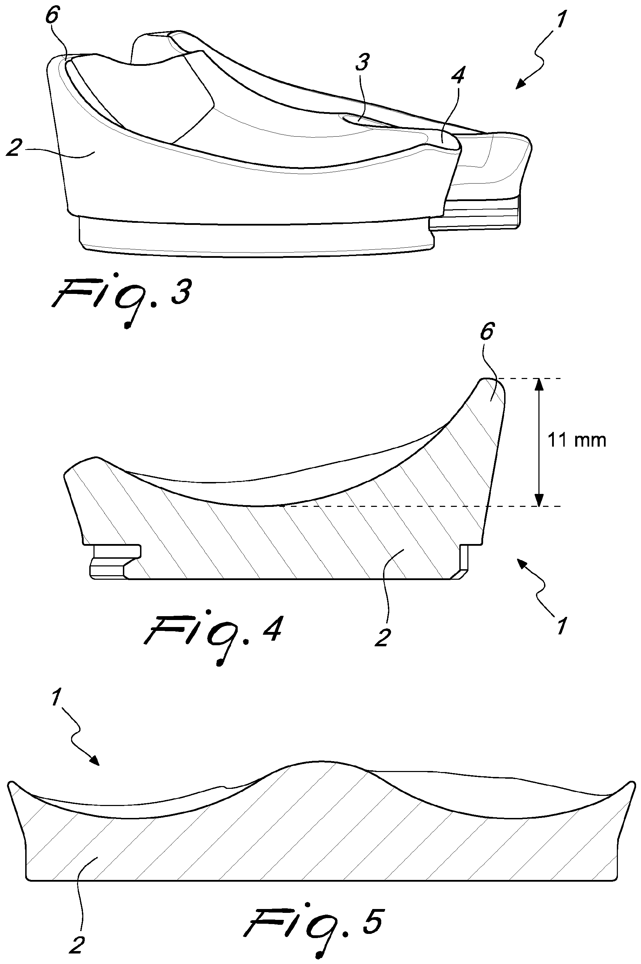

These raised portions, shown in Figure 3 and designated by the reference numeral 6, contrast the anterior dislocation of the femur and are sufficient to keep the prosthetic implant active.

Furthermore, the body 2 must have a certain shape match between the articular part of the body and the condyles of the femoral part of the artificial prosthesis, so as to assist stability.

It is also necessary to note that there are movable tibiae, in which the insert (anatomically contoured, dedicated or universal) is free to rotate on the flat portion of the tibial component of the artificial prosthesis, and fixed tibiae, in which the insert (anatomically contoured, dedicated or universal) is integral with the flat portion of the tibial component of the artificial prosthesis.

The device according to the invention must be able to interface with the movable or fixed tibiae.

In particular, the artificial knee prosthesis can be constituted by a device 1 according to the invention and by a movable tibia with a dedicated or anatomically contoured insert.

As an alternative, the artificial knee prosthesis can be constituted by a device according to the invention and by a fixed tibia with a dedicated or anatomically contoured insert.

In practice it has been found that the device according to the present invention achieves fully the intended aim and objects, since it can be used in artificial knee prostheses when one wishes to protect the posterior cruciate ligament, with the device according to the invention, which can be equally functional even in the presence of an unwanted rupture of the posterior cruciate ligament.

The device thus conceived is susceptible of numerous modifications and variations, all of which are within the scope of the appended claims.

In practice, the materials used, as well as the contingent shapes and dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device (1) for protecting the posterior cruciate ligament in knee prostheses, comprising a substantially elliptical body (2) provided with a recess (3) in its central portion, said recess (3) being provided with rounded edges (4, 5) on at least one side of the recess (3) to allow the passage of the posterior cruciate ligament, **characterized in that** a central front lip and a lateral front lip of said body (2) are of a thickness and a shape match that are sufficient to avoid dislocation even in the event of rupture of the posterior cruciate ligament, the thickness of said central front lip (6) and of said lateral front lip of said body being at least 11 mm higher than the point of lowest thickness of said body (2)

2. The device according to claim 1, **characterized in that** said recess (3) has a rounded edge (4) on the left inner side of the central portion for a device adapted to be applied to the right joint.

3. The device according to claim 1, **characterized in that** said recess (3) has a rounded edge (5) on the right inner side of the central portion for the application of the device to the left joint.

4. The device according to one or more of the preceding claims, **characterized in that** said body (2) has a shape match between the articular part of said body and the condyles of the femoral part of the artificial prosthesis.

5. The device according to one or more of the preceding claims, **characterized in that** said device is adapted to be used in combination with a tibia of the movable type.

6. The device according to one or more of the preceding claims, **characterized in that** said device is adapted to be coupled to a tibia of the fixed type.

## Patentansprüche

1. Eine Vorrichtung (1) zum Schutz des hinteren Kreuzbandes in Knieprothesen, die einen im Wesentlichen elliptischen Körper (2) umfasst, der in seinem Mittelteil mit einer Vertiefung (3) versehen ist, wobei die Vertiefung (3) auf mindestens einer Seite der Vertiefung (3) mit gerundeten Kanten (4, 5) versehen ist, um den Durchgang des hinteren Kreuzbandes zu ermöglichen; **dadurch gekennzeichnet, dass** eine zentrale Vorderlippe und eine seitliche Vorderlippe des Körpers (2) eine Dicke und passende Form haben, die ausreichen, um selbst im Falle eines Risses des hinteren Kreuzbandes eine Verlagerung zu verhindern, wobei die Dicke der zentralen Vorderlippe (6) und der seitlichen Vorderlippe des Körpers mindestens 11 mm höher sind als der Punkt geringster Dicke des Körpers (2).

2. Die Vorrichtung gemäß Anspruch 1, **dadurch gekennzeichnet, dass** die Vertiefung (3) eine gerundete Kante (4) an der linken Innenseite des Mittelteils für eine Vorrichtung hat, die ausgebildet ist, um am rechten Gelenk angebracht zu werden.

3. Die Vorrichtung gemäß Anspruch 1, **dadurch gekennzeichnet, dass** die Vertiefung (3) eine gerundete Kante (5) an der rechten Innenseite des Mittelteils für das Anbringen der Vorrichtung am linken Gelenk hat.

4. Die Vorrichtung gemäß einem oder mehreren der obigen Ansprüche, **dadurch gekennzeichnet, dass** der Körper (2) eine passende Form zwischen dem Gelenkteil des Körpers und den Kondylen des Oberschenkelteils der künstlichen Prothese hat.

5. Die Vorrichtung gemäß einem oder mehreren der obigen Ansprüche, **dadurch gekennzeichnet, dass** die Vorrichtung ausgebildet ist, um in Kombination mit einem Schienbein vom beweglichen Typ verwendet zu werden.

6. Die Vorrichtung gemäß einem oder mehreren der obigen Ansprüche, **dadurch gekennzeichnet, dass** die Vorrichtung ausgebildet ist, um mit einem Schienbein vom festen Typ gekoppelt zu werden.

## Revendications

1. Dispositif (1) pour la protection du ligament croisé postérieur dans des prothèses du genou, comportant un corps sensiblement elliptique (2) pourvu d'un évidement (3) dans sa portion centrale, ledit évidement (3) étant pourvu de bords arrondis (4, 5) sur au moins un côté de l'évidement (3) pour permettre le passage du ligament croisé postérieur, **caractérisé en ce qu'**une lèvre avant centrale et une lèvre avant latérale dudit corps (2) ont une épaisseur et une correspondance de forme qui sont suffisantes pour éviter une dislocation même dans l'éventualité d'une rupture du ligament croisé postérieur, l'épaisseur de ladite lèvre avant centrale (6) et de ladite lèvre avant latérale dudit corps étant au moins 11 mm plus élevée que le point de la plus petite épaisseur dudit corps (2).

2. Dispositif selon la revendication 1, **caractérisé en ce que** ledit évidement (3) a un bord arrondi (4) sur le côté intérieur gauche de la portion centrale pour un dispositif adapté pour être appliqué à l'articulation droite.

3. Dispositif selon la revendication 1, **caractérisé en ce que** ledit évidement (3) a un bord arrondi (5) sur le côté intérieur droit de la portion centrale pour l'application du dispositif à l'articulation gauche.

4. Dispositif selon une ou plusieurs des revendications précédentes, **caractérisé en ce que** ledit corps (2) a une correspondance de forme entre la partie articulaire dudit corps et les condyles de la partie fémorale de la prothèse artificielle.

5. Dispositif selon une ou plusieurs des revendications précédentes, **caractérisé en ce que** ledit dispositif est adapté pour être utilisé en combinaison avec un tibia du type mobile.

6. Dispositif selon une ou plusieurs des revendications précédentes, **caractérisé en ce que** ledit dispositif est adapté pour être couplé à un tibia du type fixe.
